# EUROPEAN PATENT SPECIFICATION

(11) **EP 4 613 180 B1**
(45) Date of publication and mention of the grant of the patent: **12.08.2026**
(21) Application number: 25192566.5
(22) Date of filing: 22.12.2023
(51) Int. Cl.: A61B 5/06, A61B 5/00

(54) **SYSTEMS FOR MAPPING TISSUE CONTACT VIA TRIANGULATION**
SYSTEME ZUR ABBILDUNG VON GEWEBEKONTAKT MITTELS TRIANGULATION
SYSTÈMES DE MAPPAGE DE CONTACT TISSULAIRE PAR TRIANGULATION

(30) Priority: 27.12.2022 US 202263477339 P; 10.11.2023 US 202318506666
(43) Date of publication of application: 10.09.2025
(62) Document number(s) of the earlier application(s) in accordance with Art. 76 EPC: 23219697.2 / 4 393 389
(73) Proprietor: Biosense Webster (Israel) Ltd., 2066717 Yokneam (IL)
(72) Inventor: OKARSKI, Kevin Mark, Irvine, 92618 (US); BAH, Abubakarr, Irvine, 92618 (US); PARIKH, Paras, 92618, Irvine (US)
(74) Representative: Carpmaels & Ransford LLP

(56) References cited:
- US-A1- 2019 183 378
- US-A1- 2020 205 932

## Description

### CROSS-REFERENCE TO RELATED APPLICATIONS

This application claims the benefit of prior filed U.S. Provisional Patent Application No. 63/477,339 filed on December 27, 2022.

### FIELD

The present disclosure relates generally to medical devices, and particularly to methods and systems for mapping anatomical signals in a patient body and visualizing anatomical geometries in relation to probes.

### BACKGROUND

A wide range of medical procedures involve placing objects, such as sensors, tubes, catheters, dispensing devices, and implants, within the body. In some systems, the graphical representations of anatomy and the inserted objects are displayed to assist a physician in executing a procedure. The CARTO^{®} System is an example of a system that includes location sensing technologies for determining position of an object within the body, mapping technology to build maps of anatomy (e.g. heart), and a workstation to display the graphical representations of anatomy and the inserted objects.

US2019183378A1 describes a system and method for measuring impedance across a plurality of electrodes and assessing proximity or contact between electrodes of a medical device and patient tissue. In one embodiment, contact is assessed individual electrodes and cardiac tissue using bipolar electrode complex impedance measurements. Initially, baseline impedance values are established for each of the individual electrodes based on the responses of the electrodes to the applied drive signals. After establishing the baseline impedance values a series of subsequent impedance values are measured for each electrode. For each electrode, each subsequent impedance value may be compared to a previous baseline impedance value for that electrode. If a subsequent impedance value is less than the baseline impedance value for a given electrode, the baseline impedance value may be reset to the subsequent impedance value. Such systems and method are particularly applicable to medical devices having numerous electrodes.

### SUMMARY

The invention is defined by the appended claims.

Systems and methods presented herein generally include determining that electrodes of an end effector are in contact with tissue and displaying the tissue contact graphically. Locations of electrodes of the end effector can be determined using advanced current location. Magnitude of contact between electrodes and tissue can be determined based on impedance measurements between the electrodes in contact with tissue and one or more reference electrodes (e.g. body patch(es)). Three electrodes in contact with tissue can define a plane and a vector that can be graphically displayed to indicate tissue location and orientation with respect to the end effector.

An example location-measuring system can include a medical probe, at least three electrode pads, a display, a memory, and a processor. The medical probe can include a plurality of electrodes. The at least three electrode pads can be configured to define a circuit between the each of the plurality of electrodes for measurement of impedance from each circuit. The processor can be operatively coupled to the memory, medical probe, and display. The processor can be configured to receive one or more impedance values from the plurality of electrodes in contact with a tissue of an organ, locate each of the plurality of electrodes within the organ based on triangulation by impedance measurements, select at least three electrodes having impedance values indicative of sufficient tissue contact with the organ, define a plane between the at least three electrodes, determine a centroid of the plane based at least in part on the locations of the at least three electrodes in the organ, and display a visual representation of the at least three electrodes connected by the plane and the centroid of the plane with respect to an anatomical representation of the organ.

The processor can further be configured to display an arrow extending from the centroid with respect to the anatomical representation of the organ.

Vertices of the plane can be defined by a central point on the plurality of electrodes.

The processor can further be configured to determine a center of mass for the at least three electrodes based on the respective impedance values of the at least three electrodes. The center of mass can be represented by a line orthogonal to the plane. The processor can further be configured to display a contact vector intersecting the orthogonal line.

The processor can further be configured to determine a spatial relationship between the plurality of electrodes and the at least three electrode pads based on a difference in impedance values from the at least three electrodes in contact with the tissue of the organ. The processor can further be configured to estimate, by the plane and the spatial relationship, a contact vector indicative of a magnitude of force and a direction of force of the medical probe against the tissue of the organ.

The one or more impedance values can be indicative of a magnitude of force of a respective electrode in contact with the tissue of the organ.

The contact vector can be aligned approximately central within the plane.

The spatial relationship between the plurality of electrodes and the at least three electrode pads can be indicative of an applied force between the at least three electrodes and the tissue of the organ.

The medical probe can further include an expandable basket assembly having a plurality of spines configured to bow radially outward from a collapsed configuration to an expanded configuration. The basket assembly can be configured to deform when at least a portion of the medical probe is in contact with the tissue.

The processor can be configured to adapt a contact vector based on a change in spatial relationship when the basket assembly undergoes deformation during contact with the tissue.

The processor can further be configured to display a visual representation of a contact vector in relation to the tissue of the organ.

The processor can further be configured to locate the medical probe based on a magnetic sensor disposed proximate the probe and based on a plurality of reference electromagnetic (EM) sensors. The reference EM sensors can define an EM coordinate system and a body coordinate system.

An example method for providing visual indicators of electrode contact to tissue of an organ by a catheter end effector having a plurality of electrodes can include the following steps, performed in a variety of orders, and with interleaving steps. The method can include determining location of at least a portion of the plurality of electrodes based at least in part on impedance measurements between a respective electrode and ground pads, selecting at least three electrodes of the plurality of electrodes that are in contact with the tissue, defining a plane contiguous to the at least three electrodes in contact with the tissue, and displaying a visual indicator of the plane with respect to a visual representation of the organ.

The method can further include identifying the at least three electrodes in contact with the tissue based at least in part on the impedance measurements between the respective electrode and ground pads.

The method can further include determining a centroid of the plane based on locations of the at least three electrodes in contact with the tissue.

The method can further include determining a center of mass for the at least three electrodes in contact with tissue, the center of mass being based on the respective impedance values of the at least three electrodes. The method can further include displaying a line orthogonal to the plane, the line representing the center of mass. The method can further include displaying a contact vector intersecting the orthogonal line. The contact vector can be aligned approximately central within the plane.

The method can further include orienting the medical probe based on a magnetic sensor disposed proximate the electrodes and a plurality of external reference electromagnetic (EM) sensors, the reference EM sensors defining an EM coordinate system and a body coordinate system.

The medical probe can include an expandable basket assembly having a plurality of spines extending along a longitudinal axis and converging at a central spine intersection. Each spine of the plurality of spines can having at least two electrodes thereon.

The method can further include configuring a point of each electrode as a vertex for the plane formed between the respective electrodes in contact with the tissue.

The method can further include aligning the contact vector approximately orthogonal to the plane.

The method can further include aligning the contact vector approximately central in the plane.

The method can further include adapting the contact vector based on a change in spatial relationship when the medical probe undergoes deformation during contact with the tissue of the organ.

The method can further include delivering, via the electrodes in contact with the tissue, electrical pulses for irreversible electroporation, the electrical pulses having a peak voltage of at least 900 volts (V).

The method can further include delivering, via spray ports, an irrigation fluid to the electrodes.

An example method for displaying visual representation of electrodes of a catheter in relation to an organ can include the following steps performed in a variety of orders and including interleaving steps. The method can include receiving one or more impedance values from a plurality of electrodes at a distal end of the catheter and in contact with a tissue of the organ. The method can include locating each of the plurality of electrodes within the organ based on triangulation by impedance measurements. The method can include selecting at least three electrodes having impedance values indicative of sufficient tissue contact with the organ. The method can include defining a plane between the at least three electrodes. The method can include determining a centroid of the plane based at least in part on the locations of the at least three electrodes in the organ. The method can include displaying on a screen a visual representation of the at least three electrodes connected by the plane and the centroid of the plane with respect to an anatomical representation of the organ.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1 is an illustration of an example catheter-based electrophysiology mapping and ablation system according to aspects of the present invention.
FIG. 2A is an illustration of an end effector defining triangular planes between electrodes according to aspects of the present invention.
FIG. 2B is an illustration of the end effector illustrated in FIG. 2A in a collapsed configuration according to aspects of the present invention.
FIG. 3 is an illustration of an end effector defining a triangular plane and vector according to aspects of the present invention.
FIG. 4A is an illustration of a graphical representation of anatomy, a medical probe, and a plane and a vector indicating tissue location and orientation of contacted tissue according to aspects of the present invention.
FIG. 4B is an illustration of a first example plane and vector determined by impedance according to aspects of the present invention.
FIG. 4C is an illustration of a second example plane and vector determined by impedance according to aspects of the present invention.
FIG. 4D is an illustration of a third example plane and vector determined by impedance according to aspects of the present invention.
FIG. 5 is a flow diagram of a method for providing visual indicators of electrode contact to tissue of an organ by a catheter end effector having a plurality of electrodes according to aspects of the present invention.
FIG. 6 is a flow diagram of a method for displaying visual representation of electrodes of a catheter in relation to an organ according to aspects of the present invention.

### DETAILED DESCRIPTION

The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%.

As used herein, the terms "patient," "host," "user," and "subject" refer to any human or animal subject and are not intended to limit the systems or methods to human use, although use of the subject invention in a human patient represents a preferred embodiment. In addition, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As used herein, the term "proximal" indicates a location closer to the operator or physician whereas "distal" indicates a location further away to the operator or physician.

As used herein, "operator" can include a doctor, surgeon, technician, scientist, or any other individual or delivery instrumentation associated with delivery of a multi-electrode catheter for the treatment of drug refractory atrial fibrillation to a subject.

As used herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation, including thermal ablation, as understood by a person skilled in the relevant art.

As discussed herein, the terms "bipolar", "unipolar", and "monopolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" and "monopolar" are used interchangeably herein to refer to ablation scheme utilizing a current path between two electrodes where one electrode including a high current density and high electric flux density is positioned at a treatment site, and a second electrode including comparatively lower current density and lower electric flux density is positioned remotely from the treatment site.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The term "temperature rating", as used herein, is defined as the maximum continuous temperature that a component can withstand during its lifetime without causing thermal damage, such as melting or thermal degradation (e.g., charring and crumbling) of the component.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the pertinent art in view of the teachings herein.

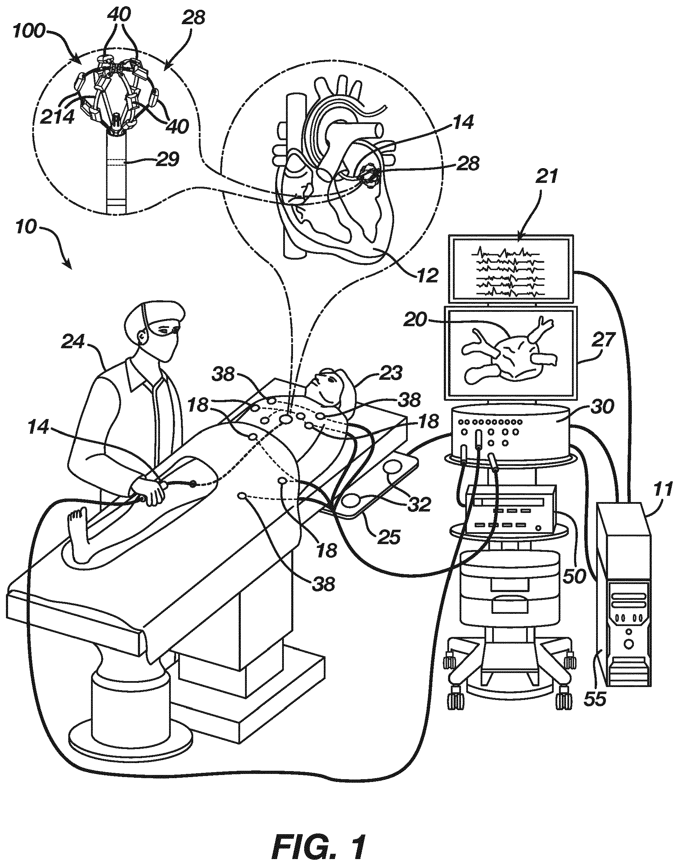

FIG. 1 is an illustration showing an example catheter-based electrophysiology mapping and ablation system 10. The system 10 includes multiple catheters, which are percutaneously inserted by a physician 24 through the patient's vascular system into a chamber or vascular structure of a heart 12. Typically, a delivery sheath catheter is inserted into the left or right atrium near a desired location in the heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM and ablation is illustrated herein. The physician 24 brings a distal tip 28 of the catheter 14 into contact with the heart wall for sensing a target site in the heart 12.

The illustrated catheter 14 is an exemplary catheter that includes one and preferably multiple electrodes 40 optionally distributed over a plurality of spines 214 of a basket assembly 100 at distal tip 28 and configured to sense the IEGM signals and provide ablation signals. Catheter 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation.

A magnetic based position sensor 29 may be operated together with a location pad 25 including a plurality of magnetic coils 32 configured to generate magnetic fields in a predefined working volume. Real time position of a distal tip 28 of the catheter 14 may be tracked based on magnetic fields generated with a location pad 25 and sensed by a magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; and 6,892,091 attached to the Appendix of priority patent Application No. 63/477,339.

The system 10 includes one or more electrode patches 38 positioned for skin contact on the patient 23 to establish location reference for location pad 25 as well as impedance-based tracking of electrodes 40. For impedance-based tracking, electrical current is directed toward electrodes 40 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182 attached to the Appendix of priority patent Application No. 63/477,339.

The magnetic based position sensor 29 can be used to calibrate impedance-based tracking of the electrodes 40. The workstation 55 can be configured to locate the distal tip 28 of the catheter 14 based on the magnetic sensor 29 and a plurality of reference electromagnetic (EM) sensors. The reference EM sensors can be configured to define an EM coordinate system and a body coordinate system.

A recorder 11 displays electrograms 21 captured with body surface ECG electrodes 18 and intracardiac electrograms (IEGM) captured with electrodes 40 of the catheter 14. The recorder 11 may include pacing capability for pacing the heart rhythm and/or may be electrically connected to a standalone pacer.

The system 10 can include an ablation energy generator 50 that is adapted to conduct ablative energy to one or more of electrodes at a distal tip of a catheter configured for ablating. Energy produced by the ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

A patient interface unit (PIU) 30 is an interface configured to establish electrical communication between catheters, electrophysiological equipment, power supply and a workstation 55 for controlling operation of system 10. Electrophysiological equipment of the system 10 may include for example, multiple catheters, a location pad 25, body surface ECG electrodes 18, electrode patches 38, an ablation energy generator 50, and a recorder 11. Optionally and preferably, the PIU 30 includes processing capability for implementing real-time computations of location of the catheters and for performing ECG calculations.

The workstation 55 includes memory, processor unit with memory or storage with appropriate operating software loaded therein, and user interface capability. The workstation 55 can be configured to provide multiple functions, optionally including (1) modeling the endocardial anatomy in three-dimensions (3D) and rendering the model or an anatomical map 20 for display on a display device 27; (2) displaying on the display device 27 activation sequences (or other data) compiled from recorded electrograms 21 in representative visual indicia or imagery superimposed on the rendered anatomical map 20; (3) displaying real-time location and orientation of multiple catheters within the heart chamber; and (4) displaying on the display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

The workstation 55 can further be adapted to detection tissue contact using impedance measurements. Details of impedance-based tissue contact, based on the impedance between the catheter electrode 40 and a return electrode 38, are described in U.S. Pat. No. 5,935,079; U.S. Pat. No. 5,836,990; U.S. Pat. No. 5,447,529; and U.S. Pat. No. 6,569,160, attached to the Appendix of priority patent Application No. 63/477,339.

The workstation 55 can further be adapted to detect tissue contact by an electrode 40 by monitoring electrical currents between electrode patches 38 and the electrode 40. Details of current-based tissue contact detection are described in US Patent No. 7,848,787 attached to the Appendix of priority patent Application No. 63/477,339. When the distal end 28 of the catheter 14 moves, the relative impedance with respect to at least one patch 38 changes. The measurement of the change in relative impedance thereby permits tracking of the distal end 28. By contrast, when catheter electrode 40 touches internal tissue, the currents at the patches 38 will change, but the values of relative impedance will not change. Consequently, as noted above, errors in position measurement due to tissue contact are reduced when the methods described above are used. These methods further provide a means of evaluating internal tissue contact by sensing when changes in current are not reflected by changes in the relative impedances.

The workstation 55 can further be adapted to detect that at least three of the electrodes 40 of the distal tip 28 of the catheter 14 that are in contact with tissue, and display, on the display 27, a visual representation of the at least three electrodes connected by a plane with respect to an anatomical representation of the heart 12. The workstation can further display a centroid of the plane with respect to the anatomical representation of the heart 12. In order to determine which electrodes 40 are in contact with tissue, the workstation 55 can be configured to receive one or more impedance values from the electrode 40 that are in contact with tissue of the heart 12, and select at least three electrodes 40 having impedance values indicative of sufficient tissue contact with the heart 12. In order to determine the spatial relationship of the electrodes 40 within the heart 12, the workstations can be configured to locate at least a portion of the electrodes 40 based on triangulation by impedance measurements. The workstation 55 can be configured to define the plane between the at least three electrodes 40 and the centroid of the plane based at least in part on the locations of the at least three electrodes that are in contact with tissue in the heart 12.

The system 10 can further include an irrigation source (not illustrated) configured to provide irrigation fluid to the catheter 14. The workstation 55 can be configured to control the irrigation source to provide irrigation at the distal end 28 of the catheter 14.

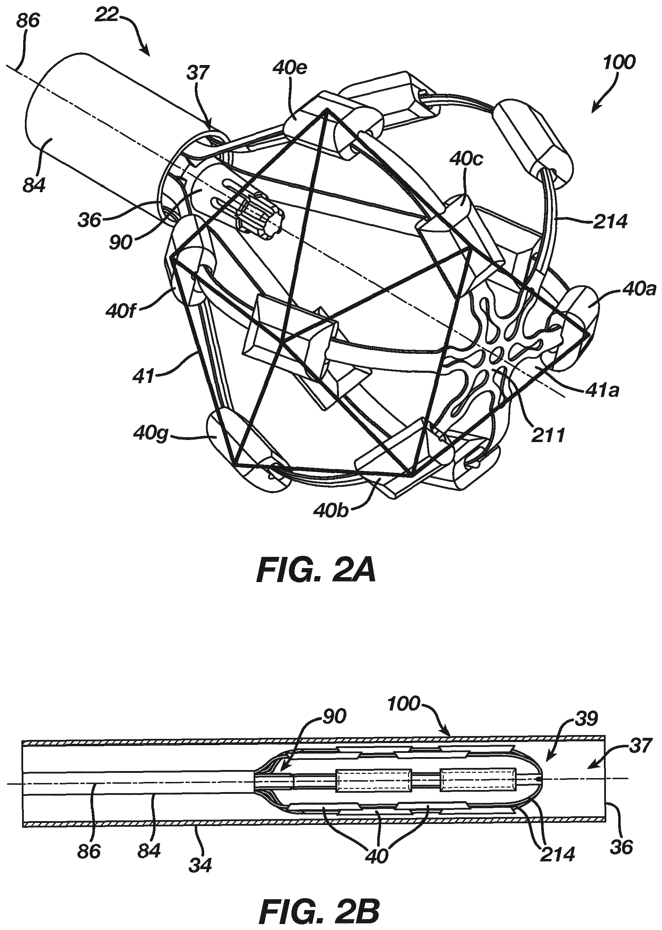

FIG. 2A is a schematic pictorial illustration showing a perspective view of a medical probe 22 including a basket assembly 100 in an expanded form when unconstrained, such as by being advanced out of an insertion tube lumen 37 (FIG. 2B) at a distal end 36 of an insertion tube 34. The medical probe 22 illustrated in FIG. 2A can be configured similarly to the catheter 14 illustrated in FIG. 1.

FIG. 2B shows the basket assembly 100 in a collapsed form within insertion tube 34 of a guide sheath. In the expanded form (FIG. 2A), spines 214 bow radially outwardly, and in the collapsed form (FIG. 2B) the spines are arranged generally along a longitudinal axis 86 of insertion tube 34.

As shown in FIG. 2A, basket assembly 100 includes a plurality of flexible spines 214 that are formed at the end of a tubular shaft 84. During a medical procedure, medical professional 24 (FIG. 1) can deploy the basket assembly 100 by extending tubular shaft 84 from the insertion tube 34, causing the basket assembly 100 to exit the insertion tube 34 and transition to the expanded form. Spines 214 may have elliptical (e.g., circular) or rectangular (that may appear to be flat) cross-sections, and include a flexible, resilient material (e.g., a shape-memory alloy such as nickeltitanium, also known as Nitinol) forming a strut.

The plurality of flexible spines 214 converge at a central spine intersection at a distal end 211 of the basket assembly 100. In some examples, the central spine intersection 211 can include one or more cutouts that allow for bending of the spines 214 when each spine respective attachment end is connected to the spine retention hub 90.

In embodiments described herein, one or more electrodes 40 positioned on spines 214 of basket assembly 100 can be configured to deliver ablation energy (RF and/or IRE) to tissue in heart 12. Additionally, or alternatively, the electrodes 40 can also be used to determine the location of basket assembly 100 and/or to measure a physiological property such as local surface electrical potentials at respective locations on tissue in heart 12. The electrodes 40 can be biased such that a greater portion of the one or more electrodes 40 face outwardly from basket assembly 100 such that the one or more electrodes 40 deliver a greater amount of electrical energy outwardly away from the basket assembly 100 (i.e., toward the heart 12 tissue) than inwardly.

Examples of materials ideally suited for forming electrodes 40 include gold, platinum and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 12.

The medical probe 22 can include a spine retention hub 90 that extends longitudinally from a distal end of tubular shaft 84 towards distal end 211 of basket assembly 100. The system 10 can include an irrigation module that delivers irrigation fluid to basket assembly 100 through the tubular shaft 84.

FIG. 2A illustrates triangular planes 41 that each include three electrodes 40. For instance, a distal plane 41a at the distal end 211 of the basket assembly 100 is defined by three electrodes 40a, 40b, 40c nearest the distal end 211. Additional planes 41 are defined by electrode trios 40b, 40c, 40d; 40c, 40d, 40e; 40b, 40d, 40g; 40d, 40e, 40f; and 40d, 40f, 40g as illustrated.

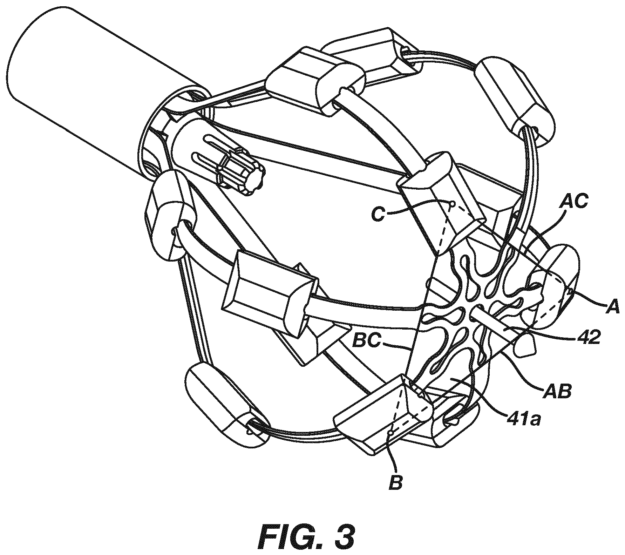

FIG. 3 illustrates features of each plane 41, using the distal plane 41a as an example. The plane 41a has three vertices A, B, C each defined by a central point of a respective electrode 40a, 40b, 40c. Each plane 41 has edges AB, BC, AC between the vertices A, B, C. FIG. 3 also illustrates a vector 42 positioned centrally within the plane 41a and pointing orthogonally from the plane 41a away from the basket assembly 100. The vector 42 can provide a visual indication of tissue location and orientation.

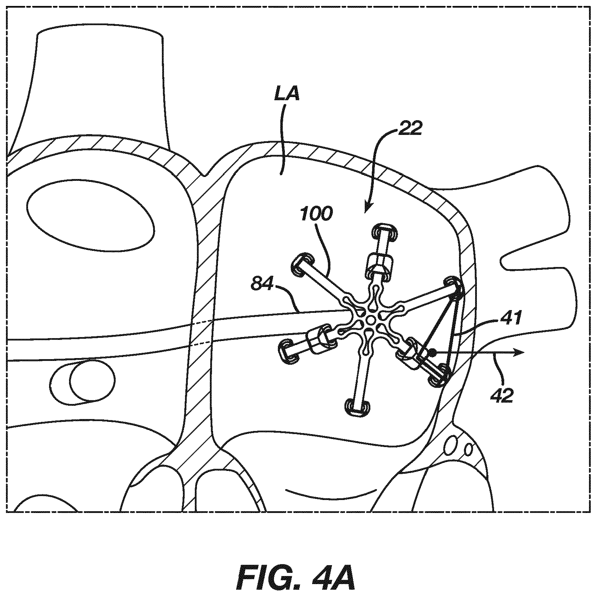

FIG. 4A is an illustration of a graphical representation of a left atrium LA of a heart, a medical probe 22, and a plane 41 and a vector 42 indicating tissue location and orientation of contacted tissue. The graphical representation can be displayed on display 27 of workstation 55 to provide a visual aid to the physician 24. Electrodes in contact with tissue can be used to create the vector 42 and the plane 41. Additionally, dynamic visualization of frame geometry (deformation) may be achieved by determining electrode positions relative to the electrode patches 38 (FIG. 1) using impedance-based location sensing, which can allow for the vector 42 to adapt to changes in frame geometry (e.g. frame deformation during contact with tissue).

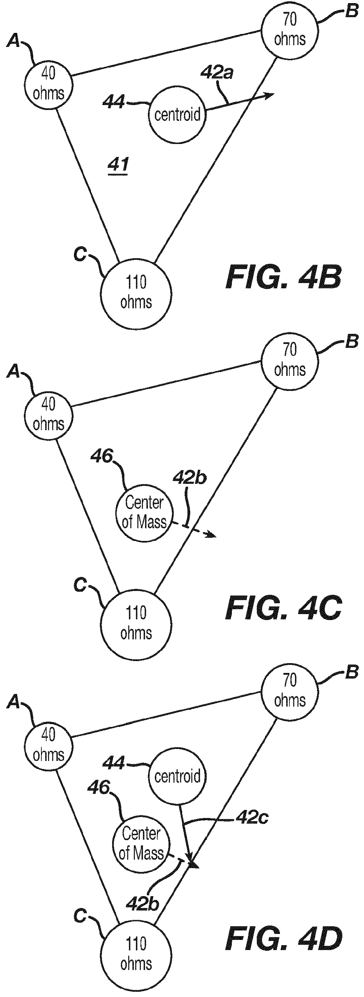

FIG. 4B is an illustration of a first example plane 41 and vector 42a determined by impedance. The locations of the three vertices A, B, C of the plane 41 can be determined by position sensing, for instance via impedance-based tracking, such as described in US Patent Nos. 7,536,218; 7,756,576; 7,848,787; 7,869,865; and 8,456,182 attached to the Appendix of priority patent Application No. 63/477,339. Additionally, or alternatively, the locations of the three vertices A, B, C of the plane 41 can be determined by magnetic based position sensing technology, such as described in U.S. Patent Nos. 5,391,199; 5,443,489; 5,558,091; 6,172,499; 6,239,724; 6,332,089; 6,484,118; 6,618,612; 6,690,963; 6,788,967; 6,892,091 attached to the Appendix of priority patent Application No. 63/477,339.

The contact impedance of each electrode at each of the three vertices A, B, C can be detected by methods as described in U.S. Pat. No. 5,935,079, U.S. Pat. No. 5,836,990, U.S. Pat. No. 5,447,529, and U.S. Pat. No. 6,569,160, attached to the Appendix of priority patent Application No. 63/477,339. In the illustrated example, vertex A has an impedance of 40 ohms, vertex B has an impedance of 70 ohms, and vertex C has an impedance of 110 ohms. The specific numerical values of impedance are selected purely for the sake of illustration and may have an alternative value consistent with the configuration of the system 10 as understood by a person skilled in the pertinent art. For the sake of the example, consider that sufficient contact is determined when the tissue contact measures at least 10 ohms. In the example, larger impedance is indicative of contact of a larger portion of surface area of the electrode 40 to tissue.

Additionally, or alternatively to the contact impedance at each vertex electrode 40, a current-based tissue contact method detection can be used, such as described in US Patent No. 7,848,787 attached to the Appendix of priority patent Application No. 63/477,339. When catheter 22 moves, the relative impedance with respect to at least one patch 38 changes. The measurement of the change in relative impedance thereby permits tracking of the catheter 22. By contrast, when catheter electrode 40 touches internal tissue, the currents at the patches 38 will change, but the values of relative impedance may not change. Consequently, errors in position measurement due to tissue contact are reduced when the methods described in US Patent No. 7,848,787 are used. These methods further provide a means of evaluating internal tissue contact by sensing when changes in current are not reflected by changes in the relative impedances. Currents at the patches 38 can therefore be used as another metric for determining tissue contact.

The vector 42a illustrated in FIG. 4B extends from a centroid 44 of the plane 41. The centroid 44 is the center point of the plane 41. Because the plane 41 is triangular, the geometrical definition of the centroid 44 of the plane 41 is the point in which the three medians of the triangle intersect. The median is a line that joins the midpoint of a side and the opposite vertex of the triangle. The centroid of the triangle separates the median in the ratio of 2:1. The x,y coordinates of the centroid 44 can be found by taking the average of x-coordinate points and y-coordinate points of all the vertices A, B, C of the triangle. The vector 42a can extend orthogonal to the plane 41, pointing toward tissue. In this example, the impedance at each vertex A, B, C is used to select the electrodes that are in contact with the tissue (e.g. electrodes having highest impedance) to define the plane 41. The vector 42a can be useful to the physician 24 to be able to visualize the central point contacted by the electrodes 40 and the direction of the tissue in relation to the electrodes 40.

FIG. 4C is an illustration of the plane 41 and a second example vector 42b determined by impedance. The plane 41 and the vertices A, B, C can be determined as disclosed in relation to FIG. 4B and elsewhere herein. A center of mass 46 of the plane can be determined based on the locations of each vertex and the relative impedance at each vertex through well-known center of mass calculations. Given that the plane is a triangle, consider that each vertex A, B, C has a respective (x,y)-coordinate (x_{A}, y_{A}), (x_{B}, y_{B}), (x_{C}, y_{C}) and the impedance represents a respective mass m_{A}, m_{B}, m_{C}, then the x-coordinate x_{CM} of the center of mass 46 can be calculated by the formula x_{CM} = (x_{A}m_{A} + x_{B}m_{B} + x_{C}m_{C}) / M, and the y-coordinate y_{CM} of the center of mass 46 can be calculated by the formula y_{CM} = (y_{A}m_{A} + y_{B}m_{B} + y_{C}m_{C}) / M, where M = m_{A} + m_{B} + m_{C}. The vector 42b can extend orthogonal to the plane 41, pointing toward tissue. The vector 42b can be useful to the physician 24 to be able to visualize a difference in contact area between the three electrodes 40 at the vertices A, B, C. The closer the center of mass 46 is to the centroid 44, the more balanced the impedance is at the vertices A, B, C, and therefore the more balanced the contact area is between the electrodes 40 at the vertices A, B, C. A physician 24 may choose to adjust the position of the basket assembly 100 to provide a desired distribution of contact between the electrodes 40. The direction of the vector 42b indicates position of tissue.

FIG. 4D is an illustration of the plane 41 and a third example vector 42c determined by impedance. The plane 41 and the vertices A, B, C can be determined as disclosed in relation to FIG. 4B and elsewhere herein. The centroid 44 can be determined as disclosed in relation to FIG. 4B. The center of mass 46 can be determined as disclosed in relation to FIG. 4C. The vector 42b from the center of mass 46 can be determined as disclosed in relation to FIG. 4C. In FIG. 4D, the center of mass vector 42b represents a line orthogonal to plane 41 that passes through the center of mass 46. A contact vector 42c extends from the centroid and intersects the orthogonal line of vector 42b rather than extending orthogonal to the plane 41. The vector 42c can be useful to the physician 24 to be able to visualize a difference in contact area between the three electrodes 40 at the vertices A, B, C. The closer the center of mass 46 is to the centroid 44, the more orthogonal the vector 42c through the centroid 44 becomes to the plane 41. The closer the center of mass 46 is to the centroid 44, the more balanced the impedance is at the vertices A, B, C, and therefore the more balanced the contact area is between the electrodes 40 at the vertices A, B, C. A physician 24 may choose to adjust the position of the basket assembly 100 to provide a desired distribution of contact between the electrodes 40.

In summary, a first vector 42a illustrated in FIG. 4B extends from the centroid 44 orthogonal to the plane 41; a second vector 42b illustrated in FIGs. 4C and 4D extends from the center of mass 46 orthogonal to the plane 41; and a third vector 42c illustrated in FIG. 4D extends from the centroid and intersects the orthogonal line of vector 42b. The plane 41 is approximately parallel to the tissue and near to the tissue to indicate location of the tissue in contact with the basket assembly 100. The vector(s) 42a, 42b, 42c therefore displayed in relation to the tissue at least by virtue of being displayed in relation to the plane 41. A graphical representation of the tissue may also be displayed.

The workstation 55 illustrated in FIG. 1 can be configured to display the first vector 42a, the second vector 42b, the third vector 42c, or any sub-combination thereof. The workstation 55 can be configured to allow the physician 24 to select which vector(s) 42a, 42b, 42c to display.

Impedance can be indicative of surface area contact of the electrodes 40 at the vertices A, B, C to tissue. A greater force applied to the electrode into tissue generally results in greater surface area contact. The workstation 55 can therefore be configured to estimate, based at least in part on geometry of the plane 41 and impedance at the vertices A, B, C a magnitude of force and a direction of force of the medical probe against the tissue of the organ. The length of a vector 42a, 42b, 42c displayed can be based at least in part on the magnitude of the force. The direction of the third vector 42c (FIG. 4D) can be based at least in part on the direction of the force.

The magnitude and/or direction of the force may additionally, or alternatively be estimated based on position of the electrodes 40 including electrodes 40 not in contact with tissue. For instance, the basket assembly 100 can have a predetermined shape that the basket assembly 100 takes when unconstrained and in free space (e.g. suspended in blood, air, or other fluid), and the basket assembly 100 can be deformed from the predetermined shape when pressed to tissue. The spatial relationship between the electrodes 40 in the predetermined shape can be determined when the basket assembly 100 is not in contact with tissue, for example through impedance-based location sensing, and/or magnetic-based location sensing, or other location techniques as understood by a person skilled in the pertinent art. Spatial relationship between electrodes 40 can again be determined when the basket assembly 100 is in contact with tissue. The mechanical properties of the basket 100 can be known such that the force and direction of force on the basket 100 due to tissue contact can be calculated based on the deformed shape of the basket 100. For instance, spring constant of the spines 214 can be known, and the force can be calculated based at least in part on the spring constant. Additionally, or alternatively, the probe 22 can be calibrated using a testing apparatus prior to treatment such that various forces are applied to the basket 100 and the deformed shape of the basket 100 is observed, and calibration information is stored in memory of the catheter 14 and/or workstation 55 so that the force can be calculated based on the calibration information and observed deformation of the basket assembly 100. The length of a vector 42a, 42b, 42c displayed can be based at least in part on the magnitude of the force. The direction of the third vector 42c (FIG. 4D) can be based at least in part on the direction of the force. The workstation 55 can be configured to dynamically adapt the displayed contact vector(s) 42a, 42b, 42c when the basket assembly 100 undergoes deformation during contact with tissue.

The foregoing methods for calculating the magnitude and/or direction of the force may eliminate the need for a contact force sensor in some applications. However, the medical probe 22 may include a contact force sensor (not illustrated) disposed inside tube 84 (FIG. 2A) and proximally in relation to basket assembly 100 and as close as possible to the basket assembly 100 so that contact with cardiac tissue by electrodes 40 can be transmitted to the contact force sensor. The contact force sensor can be used in addition to, or as an alternative to utilizing impedance at the electrodes in contact with tissue to determine contact force and therefore visual display of the contact vector(s) 42a, 42b, 42c. The contact force sensor can be used in addition to, or as an alternative to utilizing deformation of the basket 100 to determine contact force and therefore visual display of the contact vector(s) 42a, 42b, 42c. Details of an example contact force sensor are provided in U.S. Patent Application Publication No. 2021/0077180A1, which disclosure is attached to the Appendix of priority patent Application No. 63/477,339.

While the examples illustrated herein include a basket assembly 100, the principles of the systems and methods disclosed herein can be applied to various catheter geometries including other spherical catheters (e.g. balloon), planar catheters, ray catheters (e.g. PENTARAY^{®}), alternatives thereto, and variations thereof as understood by a person skilled in the pertinent art. While the example planes 41 illustrated herein are defined by three electrodes in contact with tissue, a catheter geometry having more than three electrodes in a plane can result in a displayed plane defined by more than three electrodes. A centroid and a center of mass of such a plane can be calculated based on more than three vertices as understood by a person skilled in the pertinent art. Likewise, the force magnitude and direction of a catheter end effector against tissue, and therefore the vector displayed, can be determined for alternative catheter geometries as understood by a person skilled in the pertinent art. Further electrodes 40 can have a variety of cross-sectional shapes, curvatures, lengths, etc. The illustrated electrode 40 is offered to illustrate one various configuration of electrodes 40 that can be used with the medical device 22 but should not be construed as limiting. One skilled in the art will appreciate that various other configurations of electrodes 40 can be used with the disclosed technology without departing from the scope of this disclosure. The distal end 28 of the catheter 14 can carry alternative numbers of electrodes in alternative spacings as understood by a person skilled in the pertinent art.

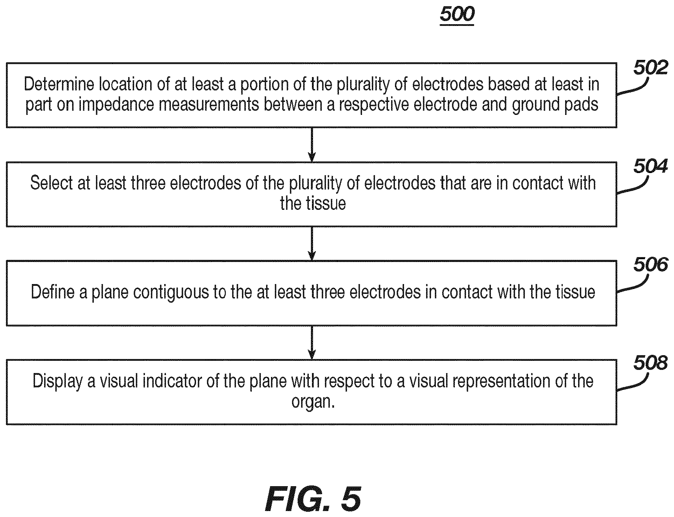

FIG. 5 is a flow diagram of a method 500 for providing visual indicators of electrode contact to tissue of an organ by a catheter end effector having a plurality of electrodes. The catheter can be configured similarly to the catheter 14 and/or medical probe 22 illustrated herein, alternatives thereto, and variations thereof as understood by a person skilled in the pertinent art. The organ can include a heart or other organ that can be accessed by catheter-based system.

At step 502, a location of at least a portion of the plurality of electrodes is determined. The location can be determined based at least in part on impedance measurements between a respective electrode and ground pads. The impedance measurements can be made using methods disclosed herein, alternatives thereto, and variations thereof as understood by a person skilled in the pertinent art. The location can be determined for electrodes in contact with tissue. Location can additionally be determined for electrodes not in contact with tissue.

At step 504, at least three electrodes that are in contact with tissue can be selected from the plurality of electrodes. The electrodes can be determined to be in contact with tissue based on methods disclosed herein, alternatives thereto, and variations thereof as understood by a person skilled in the pertinent art. For instance, at least three electrodes in contact with tissue can be identified based at least in part on impedance measurements between the respective electrode and ground pads.

At step 506, a plane contiguous to the electrodes in contact with tissue, selected at step 504, can be defined. The plane can be configured similarly to the plane 41 illustrated herein, planes otherwise disclosed herein, alternatives thereto, and variations thereof as understood by a person skilled in the pertinent art.

At step 508, a visual indicator of the plane can be displayed with respect to a visual representation of the organ. For instance, see FIG. 4A. The plane and the organ may be otherwise displayed by modifying graphical representations of the CARTO^{™} system, nGEN, alternatives thereto, and variations thereof to include a plane displayed with respect to the organ as understood by a person skilled in the pertinent art. Each electrode in contact with tissue can be configured as a vertex for the plane.

The method 500 can further include additional steps. A centroid of the plane can be determined based on locations of the electrodes in contact with tissue. A center of mass can be determined for the electrodes in contact with tissue. The center of mass can be based on the respective impedance values of the electrodes. A line orthogonal to the plane at the center of mass can be displayed. A contact vector intersecting the orthogonal line can be displayed, and may not be orthogonal to the plane. More than one contact vector can be displayed, and the vector(s) can extend from the centroid and/or the center of mass. A vector may be orthogonal to the plane. The vector can be adapted based on a change in spatial relationship when the medical probe undergoes deformation during contact with tissue of the organ. Electrical pulses can be delivered to the electrodes in contact with tissue for irreversible electroporation. The electrical pulses can have a peak voltage of at least 900 V. Irrigation fluid can be delivered via spray ports to the electrodes.

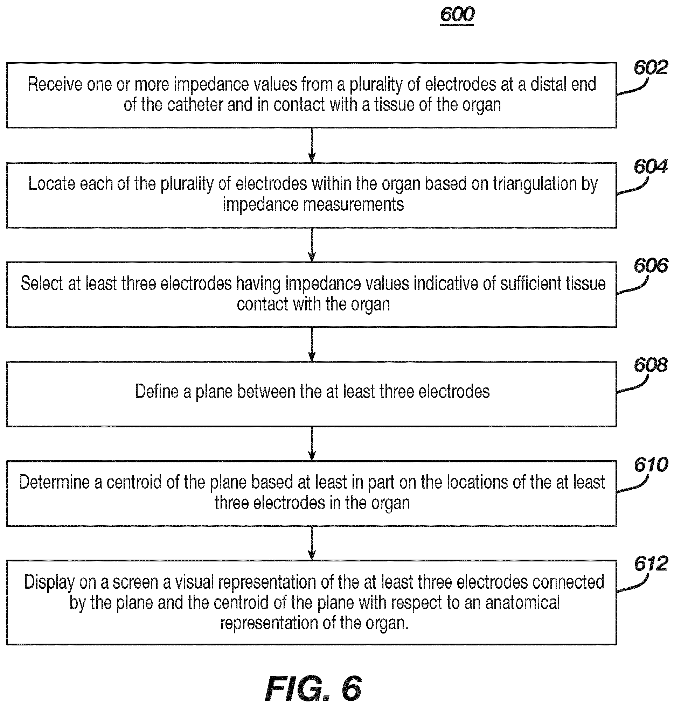

FIG. 6 is a flow diagram of a method 600 for displaying visual representation of electrodes of a catheter in relation to an organ. The catheter can be configured similarly to the catheter 14 and/or medical probe 22 illustrated herein, alternatives thereto, and variations thereof as understood by a person skilled in the pertinent art. The organ can include a heart or other organ that can be accessed by catheter-based system.

At step 602, one or more impedance values can be received from a plurality of electrodes at a distal end of the catheter and in contact with tissue of the organ. The impedance values can be determined based at least in part on impedance measurements between a respective electrode and ground pads. The impedance measurements can be made using methods disclosed herein, alternatives thereto, and variations thereof as understood by a person skilled in the pertinent art. The location can be determined for electrodes in contact with tissue.

At step 604, each of the plurality of electrodes can be located within the organ based on triangulation impedance measurements. The triangulation impedance measurements can be determined based at least in part on impedance measurements between a respective electrode and ground pads. The triangulation impedance measurements can be made using methods disclosed herein, alternatives thereto, and variations thereof as understood by a person skilled in the pertinent art. The location can be determined for electrodes in contact with tissue. Location can additionally be determined for electrodes not in contact with tissue.

At step 606, at least three electrodes that have impedance values indicative of sufficient tissue contact with the organ can be selected. The electrodes can be selected based on the impedance values being over a predetermined threshold and/or by comparing impedance values of electrodes of the catheter to each other and selecting electrodes having impedances most indicative of contact (e.g. highest impedances). The electrodes can otherwise be selected as disclosed herein, alternatives thereto, and variations thereof as understood by a person skilled in the pertinent art.

At step 608, a plane can be defined between the electrodes selected at step 606. The plane can have a vertex for at least three of the electrodes selected at step 606.

At step 610, a centroid of the plane can be determined based at least in part on the locations of the at least three electrodes in the organ.

At step 612, a visual representation of the three electrodes selected at step 606 connected by the plane defined at step 608 can be displayed on a screen with respect to an anatomical representation of the organ. The centroid of the plane determined at step 610 can be displayed.

Having shown and described exemplary embodiments of the subject matter contained herein, further adaptations of the methods and systems described herein may be accomplished by appropriate modifications without departing from the scope of the claims. In addition, where methods and steps described above indicate certain events occurring in certain order, it is intended that certain steps do not have to be performed in the order described but in any order as long as the steps allow the embodiments to function for their intended purposes. Some such modifications should be apparent to those skilled in the art. For instance, the examples, embodiments, geometrics, materials, dimensions, ratios, steps, and the like discussed above are illustrative. Accordingly, the claims should not be limited to the specific details of structure and operation set forth in the written description and drawings.

## Claims

1. A location-measuring system (10), comprising:
a medical probe comprising a plurality of electrodes (40);
at least three electrode pads (38) configured to define a circuit between the each of the plurality of electrodes for measurement of impedance from each circuit;
a display (27);
a memory; and
a processor operatively coupled to the memory, medical probe and display, the processor configured to:
receive one or more impedance values from the plurality of electrodes in contact with a tissue of an organ;
locate (502) each of the plurality of electrodes within the organ based on triangulation by impedance measurements;
select (504) at least three electrodes of the plurality of electrodes such that the at least three electrodes have impedance values indicative of sufficient tissue contact with the organ; **characterised in that** the processor is further configured to
define (506) a plane (41) between the at least three electrodes; and
display (508), on the display, a visual indicator of the plane with respect to an anatomical representation of the organ.

2. The location-measuring system of claim 1, wherein the processor is further configured to:
determine a centroid (44) of the plane based at least in part on the locations of the at least three electrodes in the organ;
display, on the display, the centroid of the plane with respect to the anatomical representation of the organ.

3. The location-measuring system of claim 2, in which the processor is further configured to:
display an arrow extending from the centroid with respect to the anatomical representation of the organ.

4. The location-measuring system according to claim 2, wherein vertices (A, B, C) of the plane are defined by a central point on the plurality of electrodes.

5. The location-measuring system of any preceding claim, in which the processor is further configured to:
determine a center of mass (46) for the at least three electrodes based on the impedance values of the at least three electrodes such that the center of mass is represented by an orthogonal line that is orthogonal to the plane; and
display a contact vector intersecting the orthogonal line.

6. The location-measuring system of any preceding claim, wherein the processor is further configured to:
determine a spatial relationship between the plurality of electrodes and the at least three electrode pads based on a difference in impedance values from the at least three electrodes in contact with the tissue of the organ; and
estimate, by the plane and the spatial relationship, a contact vector indicative of a magnitude of force and a direction of force of the medical probe against the tissue of the organ.

7. The location-measuring system of claim 6, wherein the one or more impedance values are indicative of a magnitude of force of a respective electrode in contact with the tissue of the organ.

8. The location-measuring system of claim 5, wherein the contact vector is aligned approximately central within the plane.

9. The location-measuring system of claim 6, wherein the spatial relationship is indicative of an applied force between the at least three electrodes and the tissue of the organ.

10. The location-measuring system of any preceding claim, the medical probe further comprising:
an expandable basket assembly (100) comprising a plurality of spines (214) configured to bow radially outward from a collapsed configuration to an expanded configuration.

11. The location-measuring system of claim 10, wherein the expandable basket assembly is configured to deform when at least a portion of the medical probe is in contact with the tissue.

12. The location-measuring system of claim 11, wherein the processor is configured to adapt a contact vector based on a change in spatial relationship when the expandable basket assembly undergoes deformation during contact with the tissue.

13. The location-measuring system of any preceding claim, wherein the processor is further configured to display a visual representation of a contact vector in relation to the tissue of the organ.

14. The location-measuring system of any preceding claim, wherein the processor is further configured to locate the medical probe based on a magnetic sensor disposed proximate the medical probe and a plurality of reference electromagnetic (EM) sensors, the reference EM sensors defining an EM coordinate system and a body coordinate system.

## Patentansprüche

1. Positionsmesssystem (10), umfassend:
eine medizinische Sonde, umfassend eine Vielzahl von Elektroden (40);
mindestens drei Elektrodenpads (38), die konfiguriert sind, um einen Schaltkreis zwischen jeder der Vielzahl von Elektroden zum Messen einer Impedanz von jedem Schaltkreis zu definieren;
eine Anzeige (27);
einen Speicher; und
einen Prozessor, der mit dem Speicher, der medizinischen Sonde und der Anzeige wirkgekoppelt ist, wobei der Prozessor konfiguriert ist zum:
Empfangen eines oder mehrerer Impedanzwerte von der Vielzahl von Elektroden, die mit einem Gewebe eines Organs in Kontakt sind;
Lokalisieren (502) jeder der Vielzahl von Elektroden innerhalb des Organs basierend auf einer Triangulation durch Impedanzmessungen;
derartiges Auswählen (504) von mindestens drei Elektroden der Vielzahl von Elektroden, dass die mindestens drei Elektroden Impedanzwerte aufweisen, die einen ausreichenden Gewebekontakt mit dem Organ angeben;
**dadurch gekennzeichnet, dass** der Prozessor ferner konfiguriert ist zum
Definieren (506) einer Ebene (41) zwischen den mindestens drei Elektroden; und
Anzeigen (508), auf der Anzeige, eines visuellen Indikators der Ebene in Bezug auf eine anatomische Darstellung des Organs.

2. Positionsmesssystem nach Anspruch 1, wobei der Prozessor ferner konfiguriert ist zum:
Bestimmen eines Schwerpunkts (44) der Ebene basierend mindestens teilweise auf den Positionen der mindestens drei Elektroden in dem Organ;
Anzeigen, auf der Anzeige, des Schwerpunkts der Ebene in Bezug auf die anatomische Darstellung des Organs.

3. Positionsmesssystem nach Anspruch 2, wobei der Prozessor ferner konfiguriert ist zum:
Anzeigen eines Pfeils, der sich von dem Schwerpunkt in Bezug auf die anatomische Darstellung des Organs erstreckt.

4. Positionsmesssystem nach Anspruch 2, wobei Scheitelpunkte (A, B, C) der Ebene durch einen mittleren Punkt auf der Vielzahl von Elektroden definiert sind.

5. Positionsmesssystem nach einem der vorstehenden Ansprüche, wobei der Prozessor ferner konfiguriert ist zum:
derartiges Bestimmen eines Massenmittelpunkts (46) für die mindestens drei Elektroden basierend auf den Impedanzwerten der mindestens drei Elektroden, dass der Massenmittelpunkt durch eine orthogonale Linie dargestellt wird, die orthogonal zu der Ebene ist; und
Anzeigen eines Kontaktvektors, der die orthogonale Linie schneidet.

6. Positionsmesssystem nach einem der vorstehenden Ansprüche, wobei der Prozessor ferner konfiguriert ist zum:
Bestimmen einer räumlichen Beziehung zwischen der Vielzahl von Elektroden und den mindestens drei Elektrodenpads basierend auf einer Differenz der Impedanzwerte von den mindestens drei Elektroden, die mit dem Gewebe des Organs in Kontakt sind; und
Schätzen, durch die Ebene und die räumliche Beziehung, eines Kontaktvektors, der eine Kraftgröße und eine Kraftrichtung der medizinischen Sonde auf das Gewebe des Organs angibt.

7. Positionsmesssystem nach Anspruch 6, wobei der eine oder die mehreren Impedanzwerte eine Kraftgröße einer jeweiligen Elektrode, die mit dem Gewebe des Organs in Kontakt ist, angibt.

8. Positionsmesssystem nach Anspruch 5, wobei der Kontaktvektor etwa mittig innerhalb der Ebene ausgerichtet ist.

9. Positionsmesssystem nach Anspruch 6, wobei die räumliche Beziehung eine ausgeübte Kraft zwischen den mindestens drei Elektroden und dem Gewebe des Organs angibt.

10. Positionsmesssystem nach einem der vorstehenden Ansprüche, die medizinische Sonde ferner umfassend:
eine ausdehnbare Korbanordnung (100), umfassend eine Vielzahl von Stacheln (214), die konfiguriert sind, um sich von einer zusammengeklappten Konfiguration in eine ausgedehnte Konfiguration radial nach außen zu biegen.

11. Positionsmesssystem nach Anspruch 10, wobei die ausdehnbare Korbanordnung konfiguriert ist, um sich zu verformen, wenn mindestens ein Abschnitt der medizinischen Sonde mit dem Gewebe in Kontakt ist.

12. Positionsmesssystem nach Anspruch 11, wobei der Prozessor konfiguriert ist, um einen Kontaktvektor basierend auf einer Änderung der räumlichen Beziehung anzupassen, wenn die ausdehnbare Korbanordnung während des Kontakts mit dem Gewebe eine Verformung erfährt.

13. Positionsmesssystem nach einem der vorstehenden Ansprüche, wobei der Prozessor ferner konfiguriert ist, um eine visuelle Darstellung eines Kontaktvektors in Bezug auf das Gewebe des Organs anzuzeigen.

14. Positionsmesssystem nach einem der vorstehenden Ansprüche, wobei der Prozessor ferner konfiguriert ist, um die medizinische Sonde basierend auf einem Magnetsensor, der nahe der medizinischen Sonde eingerichtet ist, und einer Vielzahl von elektromagnetischen Referenzsensoren (EM-Referenzsensoren) zu lokalisieren, wobei die EM-Referenzsensoren ein EM-Koordinatensystem und ein Körperkoordinatensystem definieren.

## Revendications

1. Système de mesure d'emplacement (10), comprenant :
une sonde médicale comprenant une pluralité d'électrodes (40) ;
au moins trois pastilles d'électrodes (38) configurées pour définir un circuit entre chacune de la pluralité d'électrodes afin de mesurer une impédance de chaque circuit ;
un dispositif d'affichage (27) ;
une mémoire ; et
un processeur couplé de manière opérationnelle à la mémoire, à la sonde médicale et au dispositif d'affichage, le processeur étant configuré pour :
recevoir une ou plusieurs valeurs d'impédance à partir de la pluralité d'électrodes en contact avec un tissu d'un organe ;
localiser (502) chacune de la pluralité d'électrodes au sein de l'organe sur la base d'une triangulation par des mesures d'impédance ;
sélectionner (504) au moins trois électrodes de la pluralité d'électrodes de telle sorte que les au moins trois électrodes ont des valeurs d'impédance indiquant un contact tissulaire suffisant avec l'organe ; **caractérisé en ce que** le processeur est en outre configuré pour
définir (506) un plan (41) entre les au moins trois électrodes ; et
afficher (508), sur le dispositif d'affichage, un indicateur visuel du plan par rapport à une représentation anatomique de l'organe.

2. Système de mesure d'emplacement selon la revendication 1, dans lequel le processeur est en outre configuré pour :
déterminer un centroïde (44) du plan sur la base, au moins en partie, des emplacements des au moins trois électrodes dans l'organe ;
afficher, sur le dispositif d'affichage, le centroïde du plan par rapport à la représentation anatomique de l'organe.

3. Système de mesure d'emplacement selon la revendication 2, dans lequel le processeur est en outre configuré pour :
afficher une flèche s'étendant à partir du centroïde par rapport à la représentation anatomique de l'organe.

4. Système de mesure d'emplacement selon la revendication 2, dans lequel des sommets (A, B, C) du plan sont définis par un point central sur la pluralité d'électrodes.

5. Système de mesure d'emplacement selon l'une quelconque revendication précédente, dans lequel le processeur est en outre configuré pour :
déterminer un centre de masse (46) pour les au moins trois électrodes sur la base des valeurs d'impédance des au moins trois électrodes, de telle sorte que le centre de masse est représenté par une ligne orthogonale qui est orthogonale au plan ; et
afficher un vecteur de contact coupant la ligne orthogonale.

6. Système de mesure d'emplacement selon l'une quelconque revendication précédente, dans lequel le processeur est en outre configuré pour :
déterminer une relation spatiale entre la pluralité d'électrodes et les au moins trois pastilles d'électrodes sur la base d'une différence de valeurs d'impédance des au moins trois électrodes en contact avec le tissu de l'organe ; et
estimer, par le plan et la relation spatiale, un vecteur de contact indiquant une amplitude de force et une direction de force de la sonde médicale contre le tissu de l'organe.

7. Système de mesure d'emplacement selon la revendication 6, dans lequel la ou les valeurs d'impédance indiquent une amplitude de force d'une électrode respective en contact avec le tissu de l'organe.

8. Système de mesure d'emplacement selon la revendication 5, dans lequel le vecteur de contact est aligné approximativement au centre du plan.

9. Système de mesure d'emplacement selon la revendication 6, dans lequel la relation spatiale indique une force appliquée entre les au moins trois électrodes et le tissu de l'organe.

10. Système de mesure d'emplacement selon l'une quelconque revendication précédente, la sonde médicale comprenant en outre :
un ensemble panier déployable (100) comprenant une pluralité de tiges (214) conçues pour se courber radialement vers l'extérieur à partir d'une configuration repliée jusqu'à une configuration déployée.

11. Système de mesure d'emplacement selon la revendication 10, dans lequel l'ensemble panier déployable est conçu pour se déformer lorsqu'au moins une partie de la sonde médicale est en contact avec le tissu.

12. Système de mesure d'emplacement selon la revendication 11, dans lequel le processeur est configuré pour adapter un vecteur de contact sur la base d'un changement de relation spatiale lorsque l'ensemble panier déployable subit une déformation pendant un contact avec le tissu.

13. Système de mesure d'emplacement selon l'une quelconque revendication précédente, dans lequel le processeur est en outre configuré pour afficher une représentation visuelle d'un vecteur de contact par rapport au tissu de l'organe.

14. Système de mesure d'emplacement selon l'une quelconque revendication précédente, dans lequel le processeur est en outre configuré pour localiser la sonde médicale sur la base d'un capteur magnétique disposé à proximité de la sonde médicale et d'une pluralité de capteurs électromagnétiques (EM) de référence, les capteurs EM de référence définissant un système de coordonnées EM et un système de coordonnées de corps.
